(19) Europäisches Patentamt
European Patent Office
Office européen des brevets

(11) Publication number : **0 470 837 A2**

(12) **EUROPEAN PATENT APPLICATION**

(21) Application number : **91307280.7**

(22) Date of filing : **08.08.91**

(51) Int. Cl.[5] : **G06F 3/033**

(30) Priority : **10.08.90 US 565483**

(43) Date of publication of application : **12.02.92 Bulletin 92/07**

(84) Designated Contracting States : **DE FR GB**

(71) Applicant : **Allen, Gregory**
**900 Ridge Road Suite F**
**Munster, Ind. 46321 (US)**

(72) Inventor : **Allen, Gregory**
**900 Ridge Road Suite F**
**Munster, Ind. 46321 (US)**

(74) Representative : **Newstead, Michael John et al**
**Page & Co. Temple Gate House Temple Gate**
**Bristol BS1 6PL (GB)**

# (54) Self-contained examination guide and storage apparatus.

(57) A computer system comprising a portable computer formed by a hand-held enclosure for a microprocessor including a memory. During use of the portable computer, a series of examination items or categories appear on one side of the enclosure. Adjacent each of the examination items is mounted an infrared transmitter. An infrared generator is sequentially connected to the transmitters and it activates the transmitters to sequentially transmit infrared signals, each of the signals being related or associated with one of the examination items. A hand-held stylus is connected to the microprocessor by a wire or tether and includes an infrared sensor. A user of the apparatus enters data in the microprocessor by scanning one of the transmitters using the stylus. The enclosure further includes a miniature tape recorder. A small microphone, which may be mounted in the stylus, is used for dictating comments during use of the apparatus. A main or central computer and a dataport connected to the main computer are also provided. The portable computer may be plugged into the dataport in order to load data from the processor of the portable computer into the main computer. The dataport also includes a display panel connected to the main computer, whereby the dataport may function as a bulletin board for displaying information, under the control of the main computer.

EP 0 470 837 A2

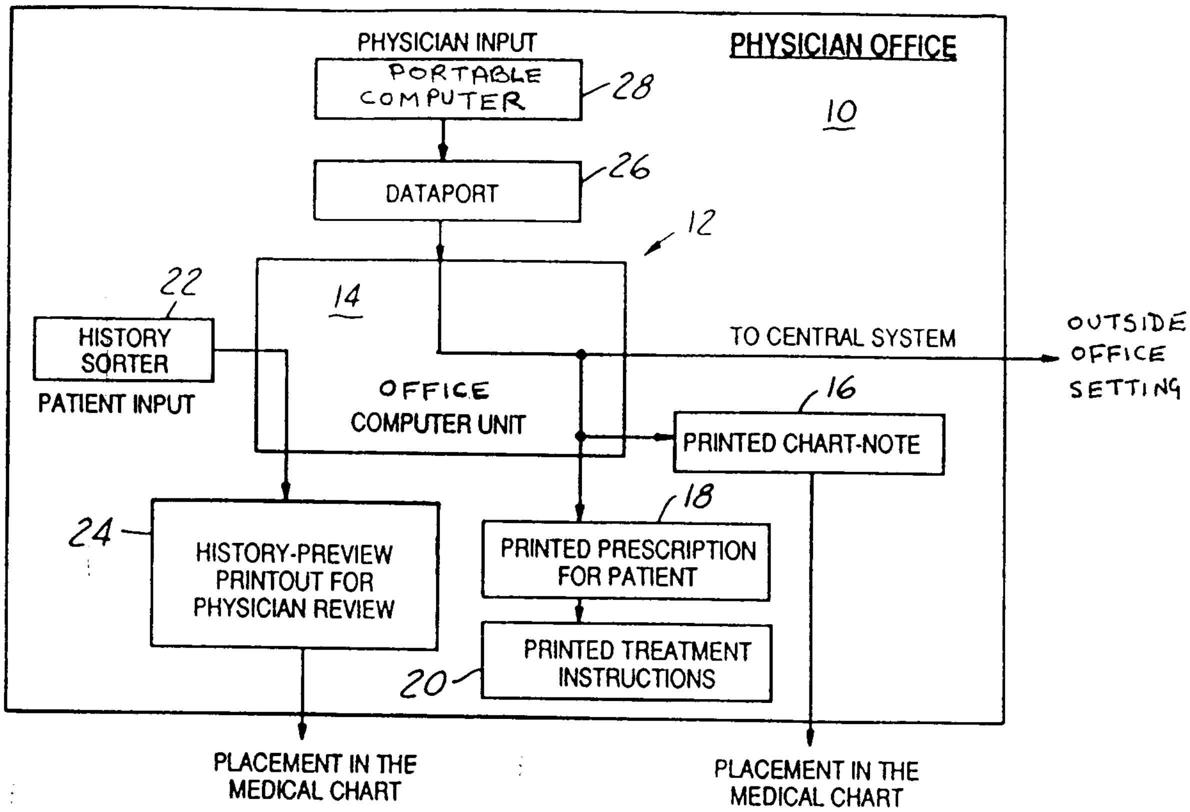
PHYSICIAN INPUT
PORTABLE COMPUTER
28
PHYSICIAN OFFICE
10
DATAPORT
26
12
14
22
HISTORY SORTER
PATIENT INPUT
OFFICE COMPUTER UNIT
TO CENTRAL SYSTEM
OUTSIDE OFFICE SETTING
16
PRINTED CHART-NOTE
18
24
HISTORY-PREVIEW PRINTOUT FOR PHYSICIAN REVIEW
PRINTED PRESCRIPTION FOR PATIENT
PRINTED TREATMENT INSTRUCTIONS
20
PLACEMENT IN THE MEDICAL CHART
PLACEMENT IN THE MEDICAL CHART

FIG. 1

## Field and Background of the Invention

This application relates to a computerized apparatus and method for assisting and/or guiding examination of a person or an article in accordance with preset categories.

Computers have become well known and are widely used in a wide variety of fields because of their exceptional capability for storing and analyzing data. Despite their wide-spread use, there are still two drawbacks or deficiencies (in addition to cost) which computer designers have strived to overcome.

One of the drawbacks has to do with size and weight. Computers were, of course, initially physically large units. The size has been gradually reduced and in recent years personal computers became possible. More recently so-called lap top computers have become available, which are portable but are normally used while sitting. Very recently a company named Grid Systems introduced a product identified as a "note-pad computer" which is small enough to be portable. It includes a display screen and a special light pen which is used to print numbers or letters on the screen. This unit apparently is small enough to be held in one hand like a clip-board while in use, while the other hand operates the pen. A note-pad type of computer is believed to be relatively expensive because of the sophisticated circuitry needed to respond to the location of the pen on the screen and the software needed to recognize handwritten characters.

The second drawback of conventional computers has been referred to as "keyboard allergy". Many people find it difficult to operate a keyboard and to learn the techniques needed to make full use of the computer's capabilities; learning to use a computer keyboard is a barrier to many. The note-pad computer discussed above is capable of recognizing printing, but the characters must be carefully drawn, and entering data by printing on a screen would be a slow method of entering data for many users.

It is a general object of the present invention to avoid the foregoing problems and to provide an improved, completely portable, self-contained computer apparatus and method for entering data and guiding an examination of a person or, article and for entering data regarding the examination. The apparatus and method are particularly useful in making a medical examination.

## Summary of the Invention

A computer apparatus in accordance with this invention comprises a portable hand-held enclosure, and a microprocessor including a memory within the enclosure. In accordance with one feature of the invention, during use of the apparatus, a series of examination items or categories appear on one side of the enclosure, preferably in a changeable display panel. Adjacent each of the examination items is mounted an infrared transmitter. An infrared generator is sequentially connected to the transmitters and it activates the transmitters to sequentially transmit infrared signals, each of the signals being related or associated with one of the examination items. A hand-held stylus is connected to the microprocessor by a wire or tether and includes an infrared sensor. A user of the apparatus enters data in the microprocessor by scanning one of the transmitters using the light pen.

In accordance with another feature of the invention, the enclosure further includes a miniature tape recorder. A small microphone, which may be mounted in the stylus, is used for dictating comments during use of the apparatus.

In accordance with still another feature of the invention, a main or central computer and a dataport connected to the main computer are provided. The portable apparatus may be glugged into the dataport in order to load data from the processor of the apparatus into the main computer. The dataport also includes a display panel connected to the main computer, whereby the dataport may function as a bulletin board for displaying information, under the control of the main computer.

## Brief Description of the Drawings

The invention will be better understood from the following detailed description taken in conjunction with the accompanying figures of the drawings, wherein:

FIG. 1 is a block diagram of an information storage and retrieval system in accordance with the present invention;

FIG. 2 is a perspective view of apparatus in accordance with the invention;

FIG. 3 is a block diagram of electronics of the apparatus of FIGS. 1 and 2;

FIG. 4 is an elevational view of an operator panel of the apparatus of FIGS. 1 to 3;

FIG. 5 is a perspective view of a portable computer apparatus in accordance with another embodiment of the invention;

FIG. 6 is a block diagram showing circuitry of the apparatus shown in FIG. 5;

FIG. 7 is a perspective view of an alternative dataport construction in accordance with this invention;

FIG. 8 is a perspective view of still another alternative dataport construction in accordance with this invention;

FIG. 9 is a block diagram of an alternate construction of the electronic components; and

FIG. 10 is a perspective view of a portable computer apparatus in accordance with a preferred embodiment of the apparatus.

Detailed Description of the Drawings

While the apparatus and method of this invention may have uses in a variety of fields, they are particularly useful in a physician's office, and the following detailed description is directed to such use.

Fig. 1 is a block diagram of a physician's office complex 10 embodying a centralized patient information storage and retrieval system 12 in accordance with this invention. System 12 includes a central computer 14 having internal memory and suitable control programming for driving a printer to generate a chart note 16 for placement in the patient file, for printing patient prescriptions 18 and corresponding treatment instructions 20, and for receiving patient history input 22 from a patient or medical technician and generating a corresponding history hardcopy 24 for placement in the patient file. In accordance with a principal feature of the present invention, central computer 14 also receives input from one or more dataports 26, which in turn are electronically coupled to corresponding portable computers 28.

For the purposes of this application, a portable computer is defined as a portable apparatus of the character illustrated in Figs. 2, 5 and 10. A dataport is defined as apparatus adapted to be connected to a central computer and to a portable computer and of the character shown in Figs. 2, 7 and 8.

As shown in Fig. 2, one embodiment of the portable computer 28 comprises a flat rectangular enclosure 30 having a front panel 32 for displaying examination information and prompting data input by the physician. Referring to both Figs. 2 and 4, the panel 32 includes a first display 34 listing general examination categories 34a-34ff along opposed side edges of panel 32. Preferably, category display 34 comprises general information diagnosis categories identified by suitable indicia permanently and legibly printed along the left-hand panel side edge--e.g., "vital signs" 34a, "appearance" 34b, "cardiovascular" 34e and "neurological" 34m. Likewise, general examination treatment categories 34r-34ff are permanently and legibly printed in suitable indicia along the right-hand edge of panel 32--e.g., "chemistry profile" 34s, "medications" 34z and "follow-up" 34ee. Immediately adjacent to each examination indicia 34a-34ff, there appears a bar code 36a-36ff for uniquely optically identifying the corresponding adjacent category. Likewise, an LED 38a-38ff appears immediately adjacent to each category indicia 34a-34ff for indicating the general examination category selected by a physician.

Centrally of panel 32, there is positioned a liquid crystal display (LCD) 40 consisting of a multiplicity of individually-controlled display segments 40a-40jj configured in two adjacent rows. As will be described in detail hereinafter, the several segments of LCD 40 display specific examination indicia coordinated with the individual general examination (diagnosis or treatment) category 34 selected by the physician. Immediately outwardly adjacent to each display segment 40a-40jj, there is positioned a corresponding bar code segment 42a-42jj for uniquely identifying the corresponding display segment, and an LED 44a-44jj for indicating to the physician the display segments selected. Along the top edge of panel 32, there is provided a central elongated LCD 46 for indicating the patient's name; indicia, bar codes and LEDs 48a-c and 50a-c for indicating "start" and "stop" of data entry respectively; and indicia, bar codes and LEDs 52a-c, 54a-c and 56a-c for indicating entry of diagnostic "history", "symptoms" and "signs" respectively. Dataport 26 (Fig. 2) comprises a generally rectangular housing 60 having an open upper edge 62 for removably receiving penboard 32, and suitable means (not shown) on the rear face for mounting housing 60 on a wall or the like.

Referring to Fig. 3, the portable computer 28 internally includes a microprocessor-based controller 64 with internal memory having prestored therein sets of specific examination indicia for display at segments 40a-40jj of LCD 40 and associated with each general examination category 34a-34ff. Controller 64 also includes suitable memory space for storing patient examination information as will be described, and control programming for operating panel 32, receiving and storing patient examination data, and subsequently transmitting or downloading such data through dataport 26 to central computer 14 (FIG. 1). Microcontroller 64 has output ports P1.0 - P1.7 connected through LED drivers 66, 68 to drive LEDs 38a-38ff and 48c-56c on panel 32. Likewise, microcontroller 64 has output ports PAO-PB7 connected through an LCD data and control driver 70 for driving LCD 46 and the several segments 40a-40jj of LCD 40. Microcontroller 64 and the remainder of the computer 28 circuitry receive power from a power supply 72 coupled to a battery 74 contained within computer enclosure 30 (Fig. 2). A reset input to microcontroller 64 is coupled to an operator pushbutton 76, and to a delay circuit 78 for resetting the microcontroller on the initial power-up.

A further input to microcontroller 64 is received from a stylus 80 that is manipulated by the physician for scanning one or more bar codes on panel 32. Stylus 80 is connected through a socket 82 (Figs. 2 and 3) on a sidewall of enclosure 30, through filtering circuitry 84 (Fig. 3) and through Schmitt triggers 86 to ports PC 6 and 7 of microcontroller 64. The input/output ports PC0 and PC3 of microcontroller 64 are coupled to a connector 88 positioned at the lower edge of computer 28, as are the transmit/receive and interrupt ports of the microcontroller 64. A mating connector 90 is internally positioned at the lower portion of dataport housing 60 for mating engagement with connector 88 when computer 28 is fully inserted into dataport 26, as shown in Fig. 2. The input/output data and control ports of microcontroller 64 are thus connected through dataport 26 to central computer 14 (Fig. 1) for downloading patient examination information and/or uploading modified control programming to the computer 28. A battery-charger 92 within dataport 26 is responsive to a switch 94 for detecting insertion of computer 28 to recharge the computer battery 74 through connectors 90, 88. As shown in Fig. 2, dataport housing 60 also includes a slot 96 at the lower edge thereof for selectively receiving computer 28 so as to mate connectors 88, 90 for downloading patient data without inserting computer 28 into dataport 26.

As previously noted, the computer 28 in the preferred embodiment of the invention has general examination category indicia 34a-34ff permanently preprinted along with the side edges of panel 32. The general category indicia illustrated in Figs. 2 and 4 represent suitable examination categories for a general practice physician. It will be noted that a number of blank spaces are provided in general information category dis-play 34 for entry of suitable general examination or treatment categories as desired by the individual practitioner. The computer 28 also has specific examination indicia prestored in microcontroller 64 coordinated with each general examination category. In addition, through central computer 14, the physician may add or modify specific examination indicia to be displayed at LCD 40. The specific indicia at display 40 illustrated in FIGS. 2 and 4 correspond to "neurologic" general examination category 34m. The following Table 1 lists computer 28 display abbreviations 40a-40jj and the corresponding text which would be printed on chart note 16 (Fig. 1) for the patient file copy:

TABLE 1

| COMPUTER SCREEN ABBREVIATION | CHART-NOTE PRINTOUTS GENERATED BY SCREEN ABBREVIATION SELECTION |
|---|---|
| CORT SEN FUNCT OBJ IDEN | CORTICAL SENSORY FUNCTION OBJECT IDENTIFICATION INTACT |
| CORT SEN FUNCT 2-POINT DISCR | CORTICAL SENSORY FUNCTION |
| OPTIC NERV | OPTIC NERVE FUNCTION INTACT |
| OCULOMOTOR NERV | OCULOMOTOR NERVE WITHOUT IMPAIRMENT |
| VISUAL FIELDS INTACT | VISUAL FIELDS BILATERALLY |
| FUNDOSCOPY | FUNDOSCOPIC EXAM SHOWS NORMAL RETINAL PATTERN |
| CORNEAL REFLX | CORNEAL REFLEX BILATERALLY INTACT |
| PUPIL RELFX, ACOM | PUPILS ACCOMMODATE WITH INTACT REACTIVITY TO LIGHT |
| 7TH CRAN NERV | NO FACIAL ASYMMETRY OR WEAKNESS DURING CONVERSATION |
| AUD VIBR SEN | AUDITORY AND VIBRATORY SENSATION |
| BILAT GAG REFLX | BILATERAL GAG REFLEX NORMAL |
| RESIS HD TURN | RESISTANCE TO HEAD TURNING DEMONSTRATES INTACT 11TH CRANIAL NERVE |
| 12TH CRAN NERV | NO ATROPHY, FASCICULATIONS, DEVIATION OF TONGUE |
| GAIT DISTUR | UNSTEADY WEAVING GAIT |
| SCOTOMAS | NEGATIVE SCOTOMA WITH CENTRAL FIELD INVOLVEMENT INTERFERRING WITH VISUAL ACUITY |

TABLE 1 (Cont'd)

| | |
|---|---|
| MUSCLE WAST | DECREASED MUSCLE BULK OUT OF PROPORTION TO WEAKNESS |
| VIBR POSIT SEN | VIBRATORY POSITION SEND INTACT |
| ATAXIA | REELING WIDE-BASED GAIT |
| DYSARTHRIA | DIMINISHED ABILITY TO PRONOUNCE WORDS WITH NO EVIDENCE OF APHASIA |
| INTEN TREM | INTENTIONAL TREMOR |
| PHYSIOL TREM | NORMAL FINE, RAPID TREMOR |
| FESTINATION | FESTINATING GAIT |
| ASTERIXIS | COARSE, SLOW, NON-RHYTHMIC TREMOR - LIVER FLAP |
| PULS EXOPHTH | PULSATING EXOPHTHALMOS |
| AUTONOM DYSFUNCT | BLOOD PRESSURE FLUCTUATIONS, CARDIAC ARRHYTHMIAS, PUPILLARY CHANGES |
| PROX LIMB WK | PROXIMAL LIMB WEAKNESS |
| LINGUAL ATROPHY | LINGUAL ATROPHY |
| PTOSIS | PTOSIS RT/LT EYELID |
| DYSPHAGIA | DYSPHAGIA |
| FASCICULATION | FASCICULATION R/O LOWER MOTOR NEURON DISEASE |
| RADIC PAIN | RADICULAR PAIN |
| SCIATICA | RADIATING RT/LEFT SCIATIC PAIN |
| INCR DP TEN REFLX | HYPERACTIVE DEEP TENDON REFLEXES |
| PROPRIO DEF | PROPRIOCEPTIVE DEFICIT |
| COGWH RIGID | COGWHELL RIDGIDTIY SUGGESTIVE OF PARKINSON'S DISEASE |
| MEIGE SYNDR | INVOLUNTARY BLINKING OF EYES AND JAW-GRINDING MOVEMENTS |

Likewise, the following Table lists penboard display abbreviations appearing at LCD 40, and corresponding chartnote text, for "cardiovascular" general examination through category 34e:

TABLE 2

| COMPUTER SCREEN ABBREVIATION | CHART-NOTE PRINTOUTS GENERATED BY SCREEN ABBREVIATION SELECTION |
|---|---|
| ATRIAL PREM BEAT | ATRIAL PREMATURE BEATS |
| VENTR PREM CONTR | VENTRICULAR PREMATURE CONTRACTION |
| ATHER COR VESSEL | ATHEROSCLEROTIC CORONARY VESSEL DISEASE |
| CONGEST FAILURE | CONGESTIVE HEART FAILURE |
| LT BUN BR BLOCK | LEFT BUNDLE BRANCH BLOCK |
| MITRAL REGUR | MITRAL VALVE REGURGITATION |
| MITRAL STENOSIS | MITRAL VALVE STENOSIS |
| APEX BEAT | APEX BEAT ENLARGE & PROLONGED IN SITTING POSITION |
| INTERMITT CLAUD | INTERMITTENT CLAUDICATION |
| PAROXY NOC DYS | PAROXYSMAL NOCTURNAL DYSPNEA |
| PUL HYPERTEN | PULMONARY HYPERTENSION |
| SICK SINUS SYNDER | SICK SINUS SYNDROME (SSS) TACHYCARDIA-BARDYCARDIA |
| SUBAORT STENOSIS | SUBAORTIC STENOSIS |
| AORTIC EJECT SD | AORTIC EJECTION SOUND |
| SYSTOL CLICK M V PROLAP | SYSTOLIC CLICK SUGGESTING MITTRAL VALVE PROLAPSE |
| SINGLE S 2 NOR VAR | SINGLE S2 - NORMAL VARIATION |
| EPIGAST PULSAT | EPIGASTRIC PULSATION |
| ORTHO DECR BP | ORTHOSTATIC DECREASED BLOOD PRESSURE |
| CAROTID PULSE | CAROTID PULSE WITHIN NORMAL LIMITS |
| PANSYST REG MUR | PANSYSTOLIC REGURGITANT MURMUR |
| EARLY DIASTOL MUR | EARLY DIASTOLIC MURMUR AT BASE AND STERNAL BORDER |

TABLE 2 (Cont'd)

| | |
|---|---|
| MID DIASTOL MUR | MID DIASTOLIC MURMUR AT APEX |
| COSTOCH TEND | TENDERNESS TO PALPATION OVER COSTOCHRONDRAL ARTICULATIONS (TIETZE'S SYNDROME) |
| LANCIN CHT PAIN | FLEETING, LANCINATING CHEST PAIN UNRELATED TO EFFORT OR EMOTIONAL EXCITEMENT, NOT INDICATING ANGINA |
| PARADOX SPLIT S2 | PARDOXICAL SPLITTING OF S2 |
| PAROXY TACH RECUR | PARAXYSMAL TACHYCARDIAS, RECURRENT R/O DIGITALIS INDUCED |
| PERICARD FRIC RUB | PERICARDIAL FRICTION RUB - AUDIBLE OVER THE PRECORDIUM, INCR. ON INSPIRATION |
| NORM 3RD SOUND | VENTRICULAR GALLOP, S3, NORMAL FOR PATIENT UNDER 30 YRS |
| CANNON SOUNDS AV DISSOC/BLK | CANNON SOUNDS SUGGESTIVE OF AV DISSOCIATION OR COMPLETE AV BLOCK |
| JUG VEN PUL | NORMAL RANGE 7-9 CM WITH A WAVE SYNCHRONOUS WITH S1 AND A |
| WAVE | SYNCHRONOUS WITH S2 |
| S1 & S2 WNL | FIRST AND SECOND HEART SOUNDS NORMAL SPLITTING |
| FIXED SPLIT S2 | FIXED SPLITTING OF S2 |
| S4 PRECD S1 LVH | S4 PRECEEDING S1, LIKELY INDICATING LEFT VENTRICULAR HYPERTROPHY |
| S3 IN DIASTROL | S3 IN DIASTOLE |
| ORTHOPNEA | ORTHOPNEA, MODERATELY SEVERE |
| INC CARD DULL | INCREASED CARDIAC DULLNESS |
| SINUS TACHY 100-150 | SINUS TACHYCARDIA 100-150 BEATS/MIN |
| ATRIAL FLUTTER 220-350 | ATRIAL FLUTTER 220-350 BEATS/ MIN |

Indicia and text for the other general examination (diagnosis and treatment) categories may be selected and/or modified as desired.

With all such information prestored in the computer 28 and computer battery 74 (Fig. 3) fully charged, the computer 28 is now ready for use by the physician in examining a patient. The examining physician first employs stylus 80 to scan a bar code or other suitable indicia on the patient file, resulting in storage of patient identification data in the portable computer's microcontroller 64 and display of patient's name at LCD 46 (Figs. 2 and 4). Assuming that the physician is examining the patient for a neurological disorder, the physician first scans bar code 36m (Fig. 4) to indicate examination in general category 34m. Specific examination indicia as shown in Fig. 4 is then displayed at LCD segments 40a-40jj, and LED 38m is illuminated to remind the physician that general examination category 34m is under study. The physician then scans bar code 48b with the stylus to indicate commencement of a data-entry process, and LED 48c is correspondingly illuminated. The physician then scans one or more bar codes 42a-42jj reflecting examination of the patient, with LEDs 44a-44jj being illuminated as appropriate. In the event that one of the specific examination indicia 40a-40jj is incorrectly selected, the physician may rescan the corresponding bar code, whereupon selection is cancelled and the LED is extinguished. When data-entry is complete, the physician scans bar code 50b and LED 50c is illuminated. The physician may then select another (diagnosis or treatment) category and repeat the specific display selection process. If the physician wishes to dictate information for the patient's file not reflected in the prestored indicia, bar code 36ff is scanned during the examination process.

When examination is complete, the computer 28 is returned to dataport 26 so that connectors 88, 90 (Fig. 3) are brought into mating engagement. The patient information stored in microcontroller 64 is then downloaded to central computer 14 (Fig. 1) for preparation of file charts, prescription forms, etc. The computer 28 may alternatively be inserted into dataport slot 96 (Fig. 2) for transmission of patient data and reset pushbutton 76 (Fig. 3) may be depressed to reset the microcontroller, whereupon computer 28 is ready for examination of a new patient. Suitable software for operating central computer 14 and microcontroller 64 in the manner described will be self-evident to the artisan.

Although the self-contained examination guide and information storage apparatus has been described in conjunction with the foregoing embodiment thereof, it will be readily appreciated that modifications may be implemented without departing from the general principles of the invention. For example, although the invention has been described in connection with the presently preferred implementation for assisting patient examination by a physician, the invention in its broadest aspects may be readily applied to other types of examination or inspection processes. For example, the portable computer 28 may be configured for inspection of automobile engines, with general inspection categories such as "carburetor", "valve timing", etc. being listed along with outside edges of the computer panel, and specific examination indicia such as "idle screw adjust" displayed at LCD 40 as a function of selected general examination category. While use of a stylus 80 aid of bar code's on the computer panel is described above, other means for entering general and specific examination information may be employed, such as membrane switches or the like positioned in place of the various bar codes adjacent to the indicia as shown in Figs. 2 and 4, and the embodiments to be described hereinafter.

Figs. 5 and 6 illustrate another construction of the portable computer, which comprises an enclosure or case 101 having a size which may be conveniently held in one hand. The computer components, including memory and auxiliary parts,shown in Fig. 6, are mounted within the enclosure 101. The enclosure includes a generally flat, rectangular front panel 102, four sides 103, and a back panel (not shown) which may be plain. A stylus 104 is connected to a tether or cord 106 which terminates in a telephone-type plug 107, the receptacle of the plug 107 being connected to the computer shown in Fig. 6. Adjacent the plug 107 is an elongated slot 108 formed in a side 103 of the enclosure 102, for storing the stylus 104 when it isn't in use. It is preferred that a plug 107 and storage slot 108 be provided on both the right and left sides of the enclosure for the convenience of both right and left-handed persons.

Two display segments 111 and 112, which are preferably backlit LCD displays, are mounted on the front panel 102. The displays 111 and 112 are, of course, connected to and under the control of the computer shown in Fig. 6. Each of the displays is substantially square in the specific example shown in Fig. 5, and rows of infrared emitters are provided adjacent the edges of each display. In this example, left and right vertical rows 113 and 114 are adjacent the display 111 and left and right vertical rows 115 and 116 are adjacent the display 112. Further, bottom rows 117 and 118 are provided adjacent the lower edges of the displays 111 and 112 respectively. Each of the rows 113 to 118 includes a plurality of infrared emitters 121. In the present specific example, each of the vertical rows includes twelve emitters and each of the bottom rows includes five emitters.

The stylus 104 has an infrared sensor or receiver 122 built into its tip. When the sensor 122 is placed on a selected transmitter 121, it will detect an infrared signal generated by the selected transmitter, and the signal will be fed to the internal computer via the cord 106.

For convenience and to conserve battery power, the tip portion (including the sensor 122) may be movably mounted on the shaft of the stylus, and a switch in the stylus may be connected to respond to movement of the tip portion. In such an arrangement, the transmitter-receiver circuits would normally be inactive, and they would be energized only when an operator presses the tip of the stylus down against a transmitter. The pressure on the stylus tip closes the above-mentioned switch and thereby energizes the circuits.

The portable computer further includes a built-in miniature tape recorder 126 (to be discussed further hereinafter) and an eject button 127. A microphone 128 is mounted in the stylus 104, and an on-off switch 129 is mounted on the shaft of the stylus 104.

The block diagram of Fig. 6 illustrates the construction and operation of the built-in computer and auxiliary circuits of the portable computer. The block 131 represents all of the infrared transmitters 121 in the six rows 113 to 118, and the block 132 represents the sensor 122 in the stylus 104. A scanner/multiplexer 133 connected to the blocks 131 and 132 serves a number of functions. A block 133 circuit is connected to the transmitters 121 and to a power supply (battery) and causes the transmitters 121 to be energized in sequence. This sequential energization occurs repetitively and all of the transmitters are sequentially energized a number of times per second. When the sensor 122 is adjacent a selected transmitter 121, it, of course, detects the signal at the time when the selected transmitter is energized, and the detected signal is fed back to the block 133. In essence, the block 133 compares the sensed signal with the position of the scanner at that instant and thereby determines the location of the stylus and the selected transmitter.

The identification of the selected transmitter 121 is passed to the block 134 which generates a keycode signal that is specific to the identified transmitter. The block 134 functions similarly to the circuit in an ordinary computer keyboard which generates a signal that is specific to a pressed key of the keyboard. The output of the block 134 is fed to a microprocessor or core computer 136 which preferably is compatible with a PC such as an IBM PC.

The core computer 136 is further connected to a memory block 137 which functions as a hard disk of a PC and stores entered information, patient information, and programs for operating the system. The block 138 represents the two displays 111 and 112; the block 139 represents a built-in rechargeable battery which powers all of the components; the block 141 represents a port for interconnecting the portable computer components with a dataport; and the block 142 represents an interface between the recorder 126 and the computer. The interface block 142 preferably includes an automatic queuing circuit connected to the core computer 136. As will be described hereinafter, during use an operator enters information using the stylus 104 and selected transmitters 121. If the operator wishes to add commentary relating to an entered piece of information, he may insert an electronic "tag" which is recorded in the memory. In the embodiment shown in Fig. 5, a tag may be formed, for example, by moving the sensor 122 a second time past the transmitter of a selected indicia. At a later time the operator dictates the commentary, which is transcribed and made a part of the record. The recorded tags thus provide a prompting framework for the dictated information; the tags reference each block of audio data to a category or indicia entered by use of the stylus.

With reference to Figs. 7 and 8, two types of dataports are shown. Fig. 8 shows a desk top dataport 151 having an inclined shelf 152 and a port (not illusttated) for connection with the port 141 of the portable computer. The dataport 151 also includes a cable (not shown) extending to a central or office main computer (the unit 14 in Fig. 1). This dataport 151 is shaped to hold the computer shown in Fig. 5 at an inclined angle so that it may be operated while positioned on a desk.

The dataport 154 shown in Fig. 7 is specially designed for wall mounting but could be used on a desk instead. It includes a slot 155 formed in its upper side, the slot 155 being sized to receive the computer. At the bottom of the slot 155 is provided a port (not shown) connected by a cable to the office computer, the port being adapted to mate with the port of the computer so that the data in the computer may be uploaded to the central office computer.

It is another feature of this invention that the dataport 154 is further provided with an LCD display 156 and a keypad 157 which are controlled by the central office computer. The display 156 may receive and display messages from the central computer and thus operate as a remote bulletin board for passing messages to a physician. The keypad 157 may be used to enter information in the computer or to call up information, for example.

Both dataports 151 and 154 are preferably also provided with means for coupling with the battery system 139 and recharging the battery.

The computer 136 may be configured or programmed to operate in a variety of ways, and the following is a preferred mode of operation. The portable computer shown in Fig. 5 is plugged into a dataport and the central computer is directed, using a conventional keyboard, to download patient information to the portable computer relating to the list of patients to be examined. The records of the patients are then stored in the computer memory within the enclosure 101. The physician removes the computer from the dataport, and an initial menu appears on one or both displays 111 and/or 112 which lists the patients whose records are in the memory. The physician makes a patient selection by moving the stylus 104 past the transmitter adjacent the selected patient's name.

After the patient selection has been made, the next screen is a list of examination categories which appears on the two displays. These categories are shown in left and right columns in each of the displays 111 and 112, the left columns being adjacent and associated with the transmitters 121 of the left rows 113 and 115 and the right columns being adjacent the right rows 114 and 116. These examination categories correspond to, for example, the categories 34 shown in Fig. 4.

The operator then selects one of the categories by pressing the sensor 122 against the transmitter adjacent the selected category. The circuit shown in Fig. 6 then functions as previously described to identify the selected transmitter and the category associated with it. As described in connection with Figs. 1 to 4, there is a list of examination indicia for each category, and after a category has been selected the next screen shows the indicia of the selected category, which appears in two columns in each display adjacent the transmitters of the four rows 113 to 116. The physician then proceeds with the examination guided by the indicia shown in the displays 111 and 112, and touches the stylus on the transmitters for selected indicia. When appropriate, comments may later be dictated and queued in the computer, as previously described.

The computer may also be programmed to show a third screen of a more condition specific indicia, which may be entered using the transmitters and the receiver as previously described.

With regard to the bottom rows 117 and 118 of transmitters, the numbers "1, 2, 3---9, 0" may appear near the bottom edge of the two displays adjacent the ten transmitters. One of the indicia may state, for example, "enter patient's temperature", or "enter pulse rate". The physician may do so by touching the transmitters of the rows 117 and 118 adjacent the appropriate numbers.

The entered data are stored in the memory 137, and the physician may follow the foregoing procedure for more than one patient. Thereafter the portable computer is inserted into a dataport and all of the data are uploaded to the central computer.

Fig. 9 illustrates an alternative circuit for energizing and responding to the infrared transmitters. A code signal generator circuit 161 generates a distinctive coded digital signal for each of the transmitters 121 (represented by the block 162). The stylus 163 senses the coded signal of one of the transmitters and feeds it through the generator 161 to a keycode generator 164 which correlates the coded signal with the selected transmitter and provides a keycode. The remainder of the construction and operation may be the same as described for Fig. 6.

Fig. 10 illustrates a preferred embodiment of the portable computer apparatus which also includes a generally rectangular box-like housing 171. Computer circuitry as illustrated in Fig. 6 is mounted within the housing 71, and a stylus 172 and a cord 173 similar to the stylus 104 and the cord 106 are connected to the computer. A recorder including a tape cassette 176 and an eject switch 177 are again provided.

Two vertically elongated, back lighted LED display panels 180 and 181 are formed on the front 182 of the housing 171, a vertically extending space 183 being provided between the two panels 180 and 181. Further, a horizontal panel 184 is provided across the top of the two panels 180 and 181, and information such as a patient's name may be displayed in the panel 184. The three panels 180, 181 and 184 may be formed by a single back lighted LED display.

Adjacent the four vertical sides of the two screens 180 and 181 are four rows 186-189 of infrared transmitters. 191 which are vertically spaced along the sides. The transmitters 191 function similarly to the transmitters 121 shown in Fig. 5, and, of course, the stylus 172 includes a receiver 192 in its tip.

As previously described, during use of the system by a physician who is examining a patient, the physician inserts the housing 171 into a dataport such as the unit shown in Fig. 7. The apparatus of Fig. 10 includes a port (not illustrated) as previously described which connects with a port in the dataport, and the records of a number of patients are loaded into the computer of the portable unit from a central computer. After removing the unit from the dataport, the patient's names are displayed on the panels 180 and 181, and the physician selects one by use of the stylus 172 and the transmitter 191 which is aligned with the selected name. The patient's identification then appears in the panel 184. Thereafer, two vertical rows of examination categories are displayed by each of the panels 180 and 181, and each category is aligned and associated with one of the transmitters 191. The physician selects a category which is appropriate for the patient being examined. Following this selection, the categories are removed and, in their place, a series of indicia appear on the panels adjacent the transmitters. The physician then examines the patient and makes appropriate entries using the stylus 172 and the transmitters 191 to enter indicia, the lines of which are adjacent the transmitters.

If the physician wishes to add further comments to the patient's record after an indicia has been marked, an electronic tag may be recorded by moving the receiver 192 past a transmitter 193 or 193a. After the examination has been completed, a "dictate" screen appears on the panels wherein all of the tagged indicia are listed adjacent the transmitters. The physician then selects the tagged indicia sequentially andcomments are dictated into a microphone 194 in the stylus 172. After the examination, the physician inserts the portable apparatus into the dataport in order to load the entered data into the central computer and to load the next series of patient's files into the portable apparatus. The tape cassette with dictated matter on it may be removed and replaced by a fresh cassette.

At any time during examination the unit may be actuated to return to a primary or top level screen by moving the receiver 192 past a home transmitter 195 or 195a. Two sets of transmitters 193, 193a, 195, 195a may be provided for the convenience of right and left-handed persons.

The use of infrared transmitters and a receiver as described herein for selecting categories and indicia at various levels is advantageous because the categories and indicia may easily be changed or expanded by modifying the program. In the apparatus of Fig. 1, the categories are printed and, of course, more difficult to change. The present invention is also advantageous compared with a light pen arrangement of a note-pad computer. A light pen requires a raster type of display screen whereas the present invention may utilize a simpler and less expensive LED display. The portable computer may be either hardware or software controlled to function as described.

## Claims

**1.** A portable examination guide comprising:
a) a portable enclosure including a panel;
b) computer means including a memory mounted in said enclosure;
c) a visual display on said panel and connected for control by said computer, said computer being programed to display lines of visual matters;
d) a plurality of infrared transmitters mounted on said panel adjacent said display, one of said transmitters being associated with each of said lines, and said transmitters being connected for control by said computer;
e) stylus means connected to said computer and including infrared sensor means responsive to infrared signals from said transmitters; and
f) said computer including component means connected to said transmitters and to said sensor means for correlating an infrared signal with a line of visual matter.

**2.** Apparatus as set forth in Claim 1, wherein said component means sequentially energizes said transmitters and correlates the time of a signal from said sensor means with the time of energization of a transmitter.

**3.** Apparatus as set forth in Claim 1, wherein said visual display includes two portions, and said transmitters are arranged in rows on opposite sides of said two portions.

**4.** Apparatus as set forth in Claim 1, wherein said stylus means includes a tip, and said sensor means is mounted in said tip.

**5.** Apparatus as set forth in Claim 4 wherein said tip is movably mounted in said stylus means, and further including a switch in said stylus means and responsive to pressure on said tip for energizing said sensor means.

**6.** Apparatus as set forth in Claim 1, wherein said stylus means is attached by a plug connector to said enclosure, and a plurality of plug means are on opposite sides of said enclosure.

**7.** Apparatus as set forth in Claim 1, wherein said component means energizes said transmitters to generate coded signals, and said sensor means is responsive to said coded signals.

**8.** A portable examination guide comprising:
a) a portable enclosure including a panel;
b) computer means including a memory mounted in said enclosure;
c) a visual display on said panel and connected for control by said computer, said computer being programed to display lines of visual matters;
d) identification means on said panel adjacent said displays, one of said identification means being associated with each of said lines, and said identification means being connected for control by said computer;
e) stylus means connected to said computer and including sensor means responsive to said identification means; and
f) recorder means mounted in said enclosure.

9. Apparatus as set forth in Claim 8, wherein said recorder means includes a microphone mounted in said stylus means.

10. Apparatus as set forth in Claim 8, and further including queuing means connected to said computer means for correlating dictated matter with a line of visual matter.

11. An examination system comprising a central computer, at least one dataport, and at least one portable examination guide, said dataport being connected to said central computer, said dataport and said guide including ports for connecting said guide to said central computer, and said dataport further comprising a visual display connected to be controlled by said central computer.

12. Apparatus as set forth in Claim 11, wherein said dataport further comprises a keypad for entering instructions in said central computer.

13. A method of conducting an examination of a subject with the aid of a portable computer having a processor-memory therein, comprising:
a) providing said processor-memory with a database including a plurality of examination categories and for each category a set of examination indicia, and generating an infrared signal for each of said categories and said indicia;
b) reviewing a subject of examination, selecting an examination category by sensing an infrared signal associated therewith, entering said selection is said processor-memory, retrieving from said processor-memory the set of examination indicia associated with said selected category, and displaying on said portable computer the set of examination indicia;
c) reviewing the subject of examination, selecting indicia from said set by sensing an infrared signal associated therewith, and entering said selected indicia in said processor-memory; and
d) downloading data from said processor-memory into a central computer.

14. A method of conducting an examination of a subject with the aid of a portable computer having a processor-memory and a tape recorder therein, comprising:
a) providing said processor-memory with a database including a plurality of examination categories and for each category a set of examination indicia, and generating an infrared signal for each of said categories and said indicia;
b) reviewing a subject of examination, selecting an examination category by sensing an infrared signal associated therewith, entering said selection is said processor-memory, retrieving from said processor-memory the set of examination indicia associated with said selected category, and displaying on said portable computer the set of examination indicia;
c) reviewing the subject of examination, selecting indicia from said set by sensing an infrared signal associated therewith, and entering said selected indicia in said processor-memory;
d) dictating comments in said tape recorder relating to a selected indicia and queuing said processor-memory to correlate said comments with said selected indicia; and
e) downloading data from said processor-memory into a central computer.

15. A method of collecting examination data and entering said data in a computer system including a central computer, at least one dataport coupled with said central computer, and at least one portable computer, the central computer including a processor-memory, the dataport being separate from the central computer but coupled with the processor-memory of the central computer and said dataport having visual display means thereon, and said portable computer including a processor-memory having stored therein a plurality of examination categories and for each category a set of examination indicia, said method comprising the steps of:
a) entering in said processor-memory of said portable computer an identification of a subject of examination;
b) selecting an examination category from said plurality and entering said selected category in said processor-memory of said portable computer;
c) entering examination indicia of said set for said selected category in said processor-memory of said portable computer;

d) operating said central computer to show information on said visual display means of said dataport; and

e) coupling said portable computer with said dataport and downloading stored data from said processor-memory of said portable computer to said processor-memory of said central computer.

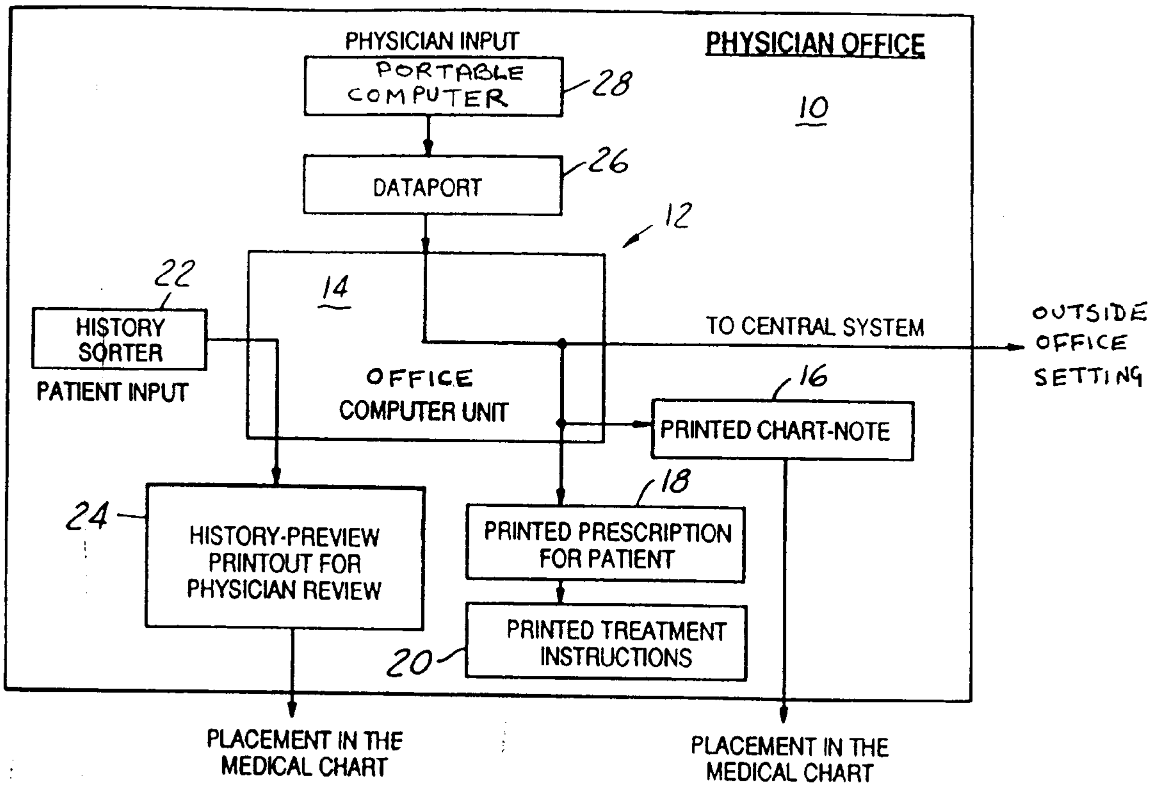
PHYSICIAN OFFICE
10
PHYSICIAN INPUT
PORTABLE COMPUTER
28
DATAPORT
26
12
14
OFFICE COMPUTER UNIT
22
HISTORY SORTER
PATIENT INPUT
TO CENTRAL SYSTEM
OUTSIDE OFFICE SETTING
16
PRINTED CHART-NOTE
18
PRINTED PRESCRIPTION FOR PATIENT
20
PRINTED TREATMENT INSTRUCTIONS
24
HISTORY-PREVIEW PRINTOUT FOR PHYSICIAN REVIEW
PLACEMENT IN THE MEDICAL CHART
PLACEMENT IN THE MEDICAL CHART

FIG. 1

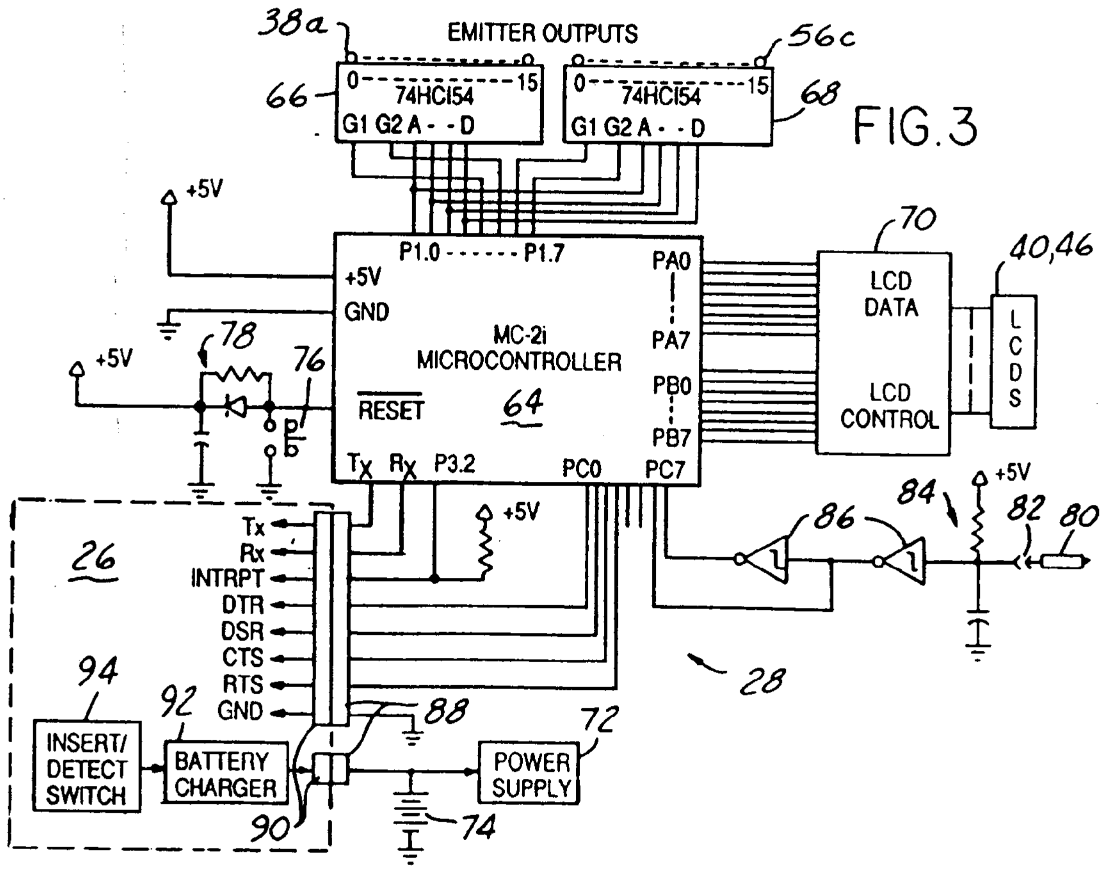
EMITTER OUTPUTS
38a
56c
66
74HC154
68
70
LCD DATA
LCD CONTROL
40,46
LCDS
+5V
GND
P1.0 - - - - - - P1.7
PA0
PA7
PB0
PB7
MC-2i MICROCONTROLLER
RESET
64
Tx Rx P3.2
PC0 PC7
78
76
26
Tx
Rx
INTRPT
DTR
DSR
CTS
RTS
GND
94
92
INSERT/ DETECT SWITCH
BATTERY CHARGER
88
90
72
POWER SUPPLY
74
86
84
82
80
28

FIG.3

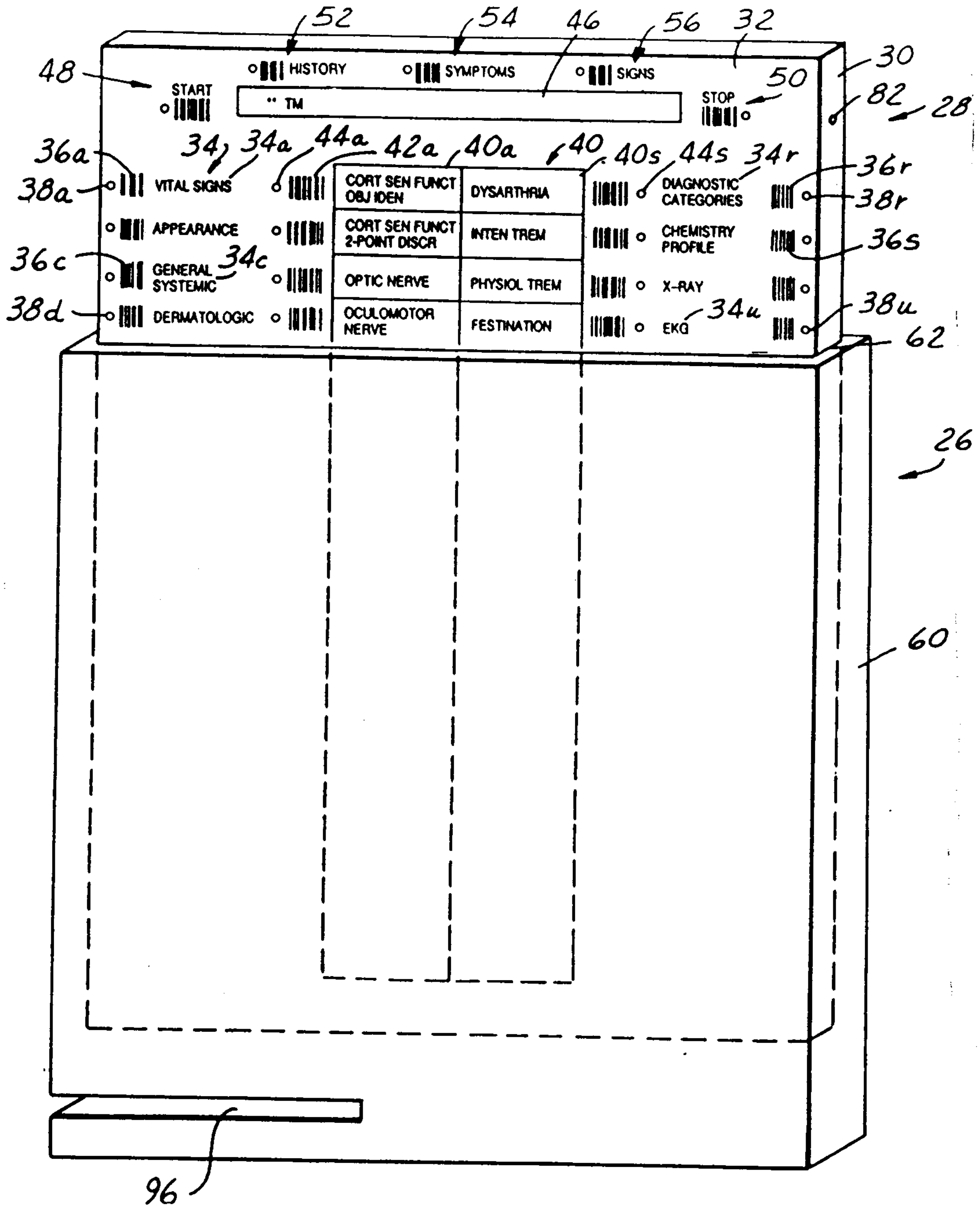

52
54
46
56
32
30
48
START
HISTORY
SYMPTOMS
SIGNS
STOP
TM
50
82
28
36a
34
34a
44a
42a
40a
40
40s
44s
34r
36r
38a
VITAL SIGNS
CORT SEN FUNCT OBJ IDEN
DYSARTHRIA
DIAGNOSTIC CATEGORIES
38r
APPEARANCE
CORT SEN FUNCT 2-POINT DISCR
INTEN TREM
CHEMISTRY PROFILE
36s
36c
GENERAL SYSTEMIC
34c
OPTIC NERVE
PHYSIOL TREM
X-RAY
38u
38d
DERMATOLOGIC
OCULOMOTOR NERVE
FESTINATION
EKG
34u
62
26
60
96


FIG.2

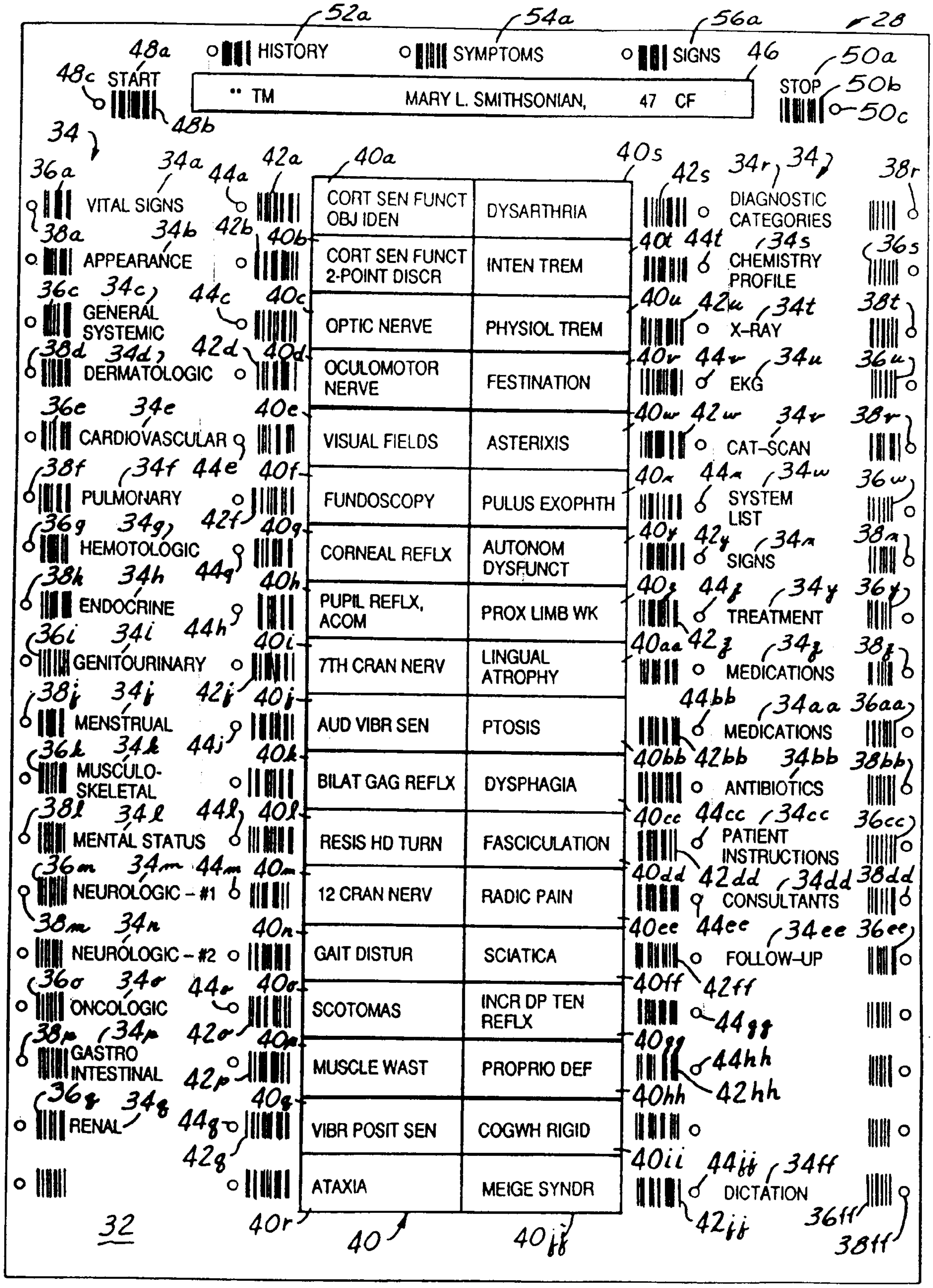
HISTORY
SYMPTOMS
SIGNS
START
STOP
•• TM MARY L. SMITHSONIAN, 47 CF
VITAL SIGNS
APPEARANCE
GENERAL SYSTEMIC
DERMATOLOGIC
CARDIOVASCULAR
PULMONARY
HEMOTOLOGIC
ENDOCRINE
GENITOURINARY
MENSTRUAL
MUSCULO-SKELETAL
MENTAL STATUS
NEUROLOGIC - #1
NEUROLOGIC - #2
ONCOLOGIC
GASTRO INTESTINAL
RENAL
CORT SEN FUNCT OBJ IDEN
DYSARTHRIA
CORT SEN FUNCT 2-POINT DISCR
INTEN TREM
OPTIC NERVE
PHYSIOL TREM
OCULOMOTOR NERVE
FESTINATION
VISUAL FIELDS
ASTERIXIS
FUNDOSCOPY
PULUS EXOPHTH
CORNEAL REFLX
AUTONOM DYSFUNCT
PUPIL REFLX, ACOM
PROX LIMB WK
7TH CRAN NERV
LINGUAL ATROPHY
AUD VIBR SEN
PTOSIS
BILAT GAG REFLX
DYSPHAGIA
RESIS HD TURN
FASCICULATION
12 CRAN NERV
RADIC PAIN
GAIT DISTUR
SCIATICA
SCOTOMAS
INCR DP TEN REFLX
MUSCLE WAST
PROPRIO DEF
VIBR POSIT SEN
COGWH RIGID
ATAXIA
MEIGE SYNDR
DIAGNOSTIC CATEGORIES
CHEMISTRY PROFILE
X-RAY
EKG
CAT-SCAN
SYSTEM LIST
SIGNS
TREATMENT
MEDICATIONS
MEDICATIONS
ANTIBIOTICS
PATIENT INSTRUCTIONS
CONSULTANTS
FOLLOW-UP
DICTATION
32


FIG.4

FIG. 5

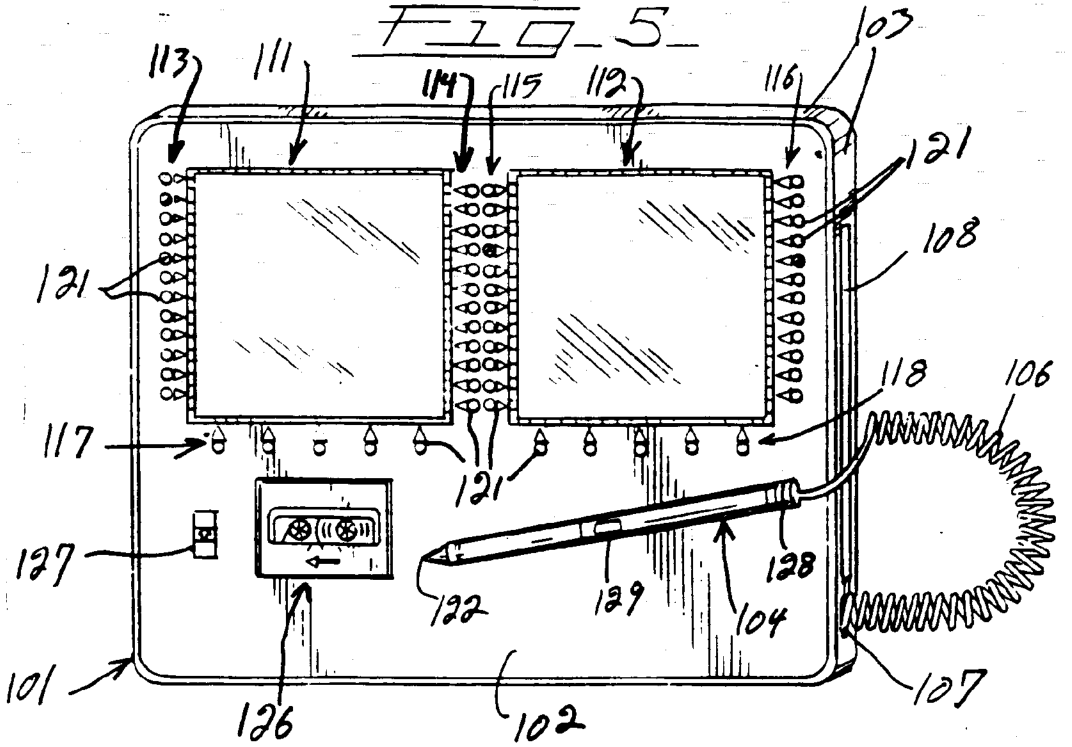
113
111
114
115
112
116
103
121
108
118
106
117
127
122
129
128
104
101
126
102
107

FIG. 6

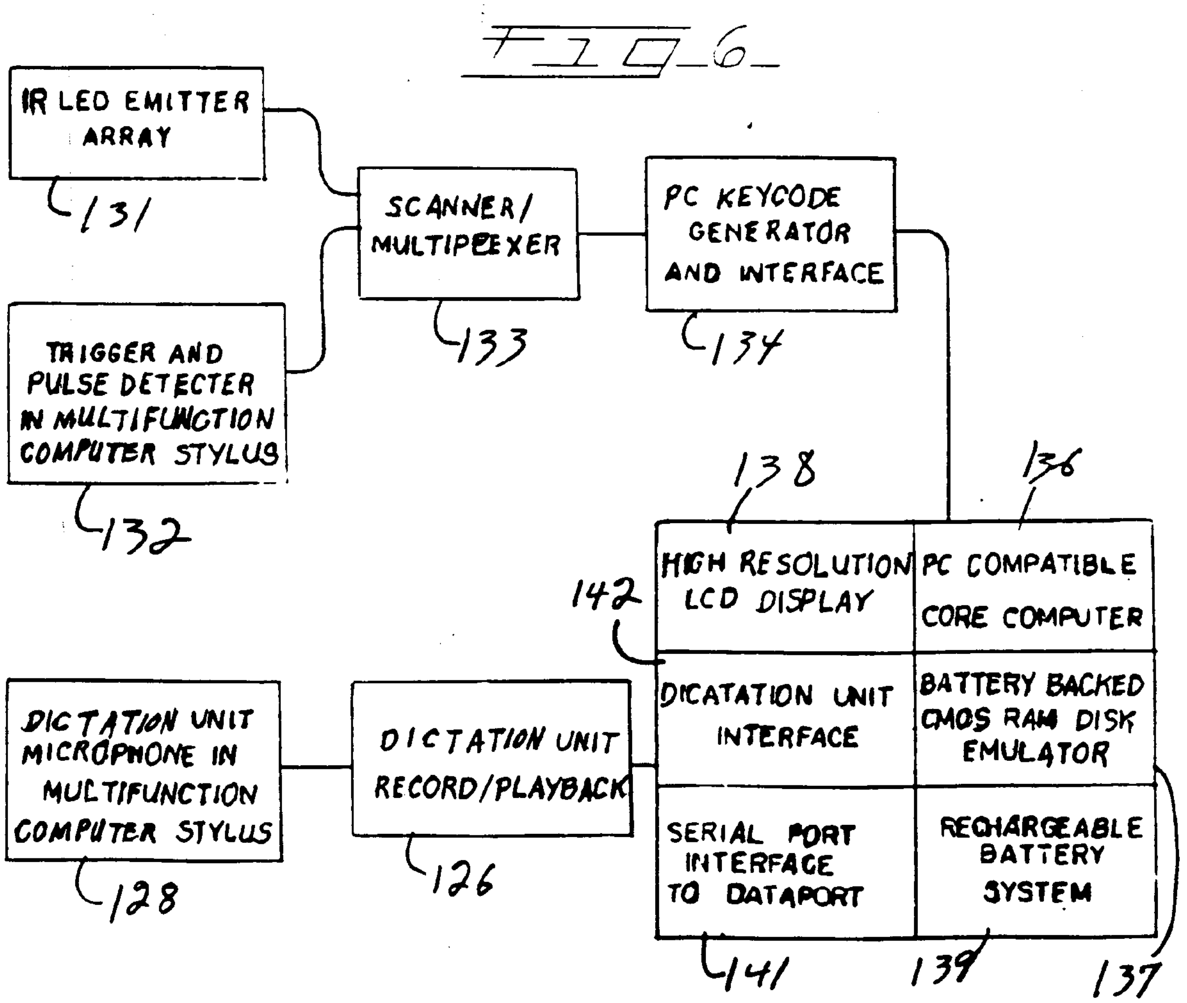
IR LED EMITTER ARRAY
131
SCANNER/ MULTIPEEXER
133
PC KEYCODE GENERATOR AND INTERFACE
134
TRIGGER AND PULSE DETECTER IN MULTIFUNCTION COMPUTER STYLUS
132
138
136
HIGH RESOLUTION LCD DISPLAY
PC COMPATIBLE CORE COMPUTER
142
DICATATION UNIT INTERFACE
BATTERY BACKED CMOS RAM DISK EMULATOR
DICTATION UNIT MICROPHONE IN MULTIFUNCTION COMPUTER STYLUS
DICTATION UNIT RECORD/PLAYBACK
SERIAL PORT INTERFACE TO DATAPORT
RECHARGEABLE BATTERY SYSTEM
128
126
141
139
137

Fig_7_

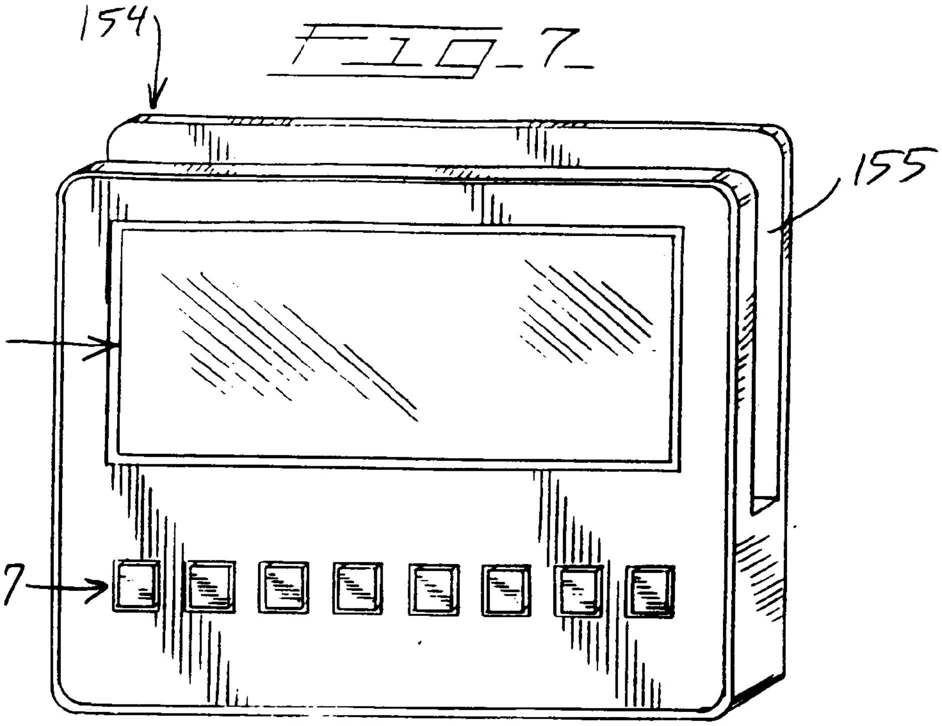
154
155
156
157

Fig_8_

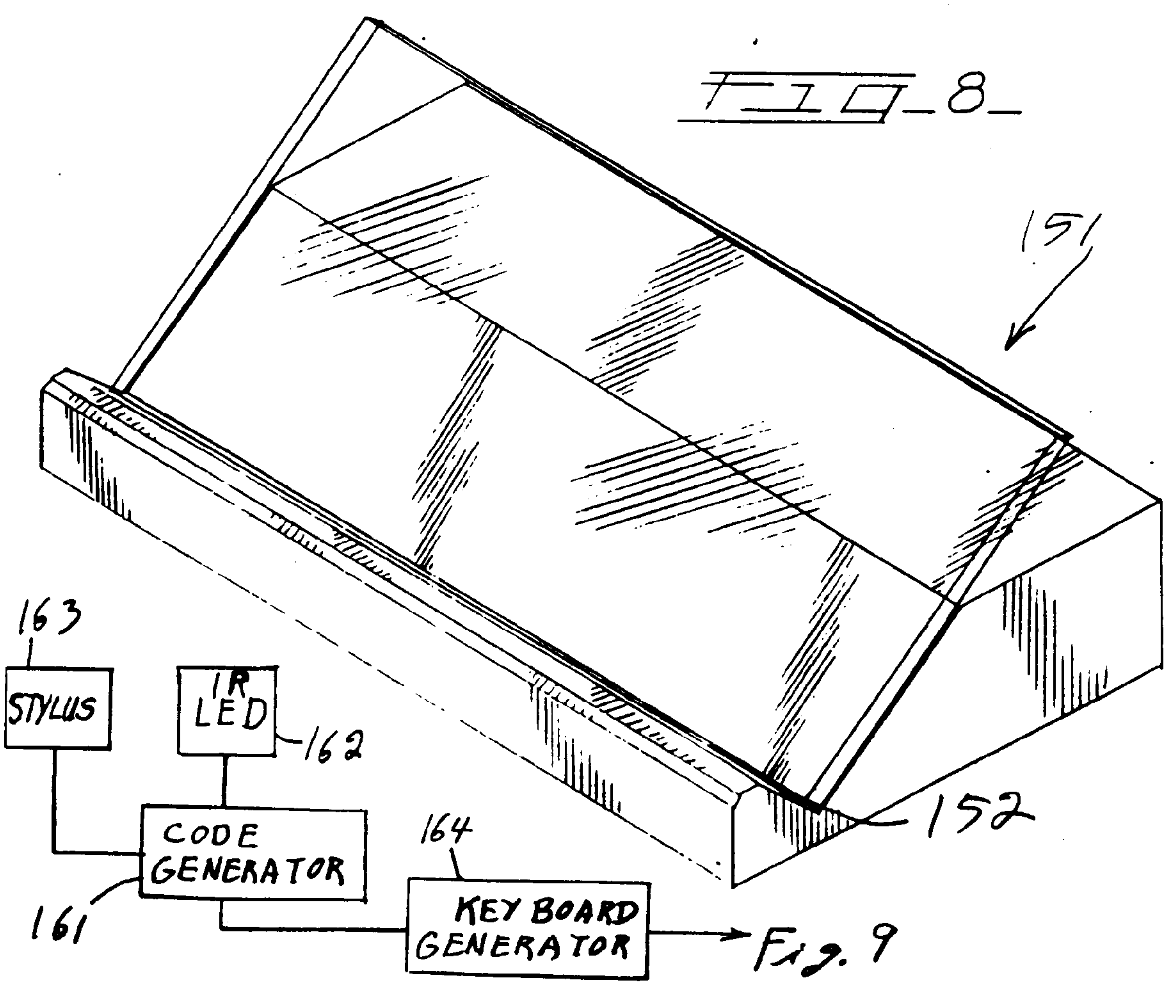
151
152
163
STYLUS
IR
LED
162
CODE
GENERATOR
161
164
KEY BOARD
GENERATOR
Fig. 9

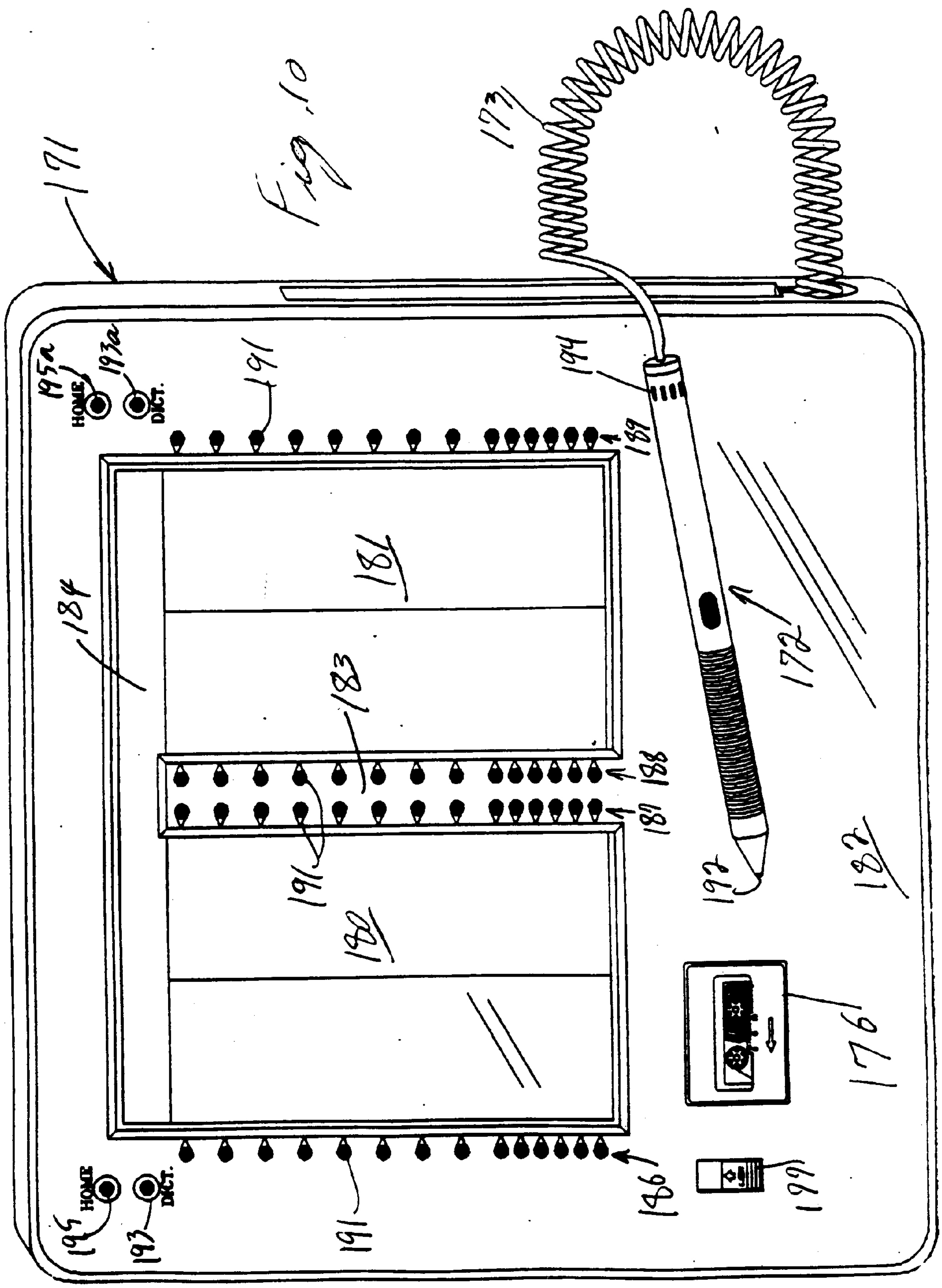
HOME
DICT.
HOME
DICT.
171
172
173
176
180
181
182
183
184
186
187
188
189
191
192
193
193a
194
195
195a
199

Fig. 10